Europäisches Patentamt

European Patent Office

Office européen des brevets

(19)

(11) Veröffentlichungsnummer : **0 063 343**
**B1**

(12)

# EUROPÄISCHE PATENTSCHRIFT

(45) Veröffentlichungstag der Patentschrift :
28.08.85

(21) Anmeldenummer : 82103081.4

(22) Anmeldetag : 09.04.82

(51) Int. Cl.⁴ : **A 61 M 5/30**

(54) **Nadelloses Injektionsgerät.**

(30) Priorität : **16.04.81 DE 3115377**

(43) Veröffentlichungstag der Anmeldung :
**27.10.82 Patentblatt 82/43**

(45) Bekanntmachung des Hinweises auf die Patenterteilung : **28.08.85 Patentblatt 85/35**

(84) Benannte Vertragsstaaten :
**CH DE FR GB LI**

(56) Entgegenhaltungen :
**US-A- 2 296 998**
**US-A- 2 653 602**
**US-A- 3 945 379**

(73) Patentinhaber : **HOECHST AKTIENGESELLSCHAFT**
**Postfach 80 03 20**
**D-6230 Frankfurt am Main 80 (DE)**

(72) Erfinder : **Dettbarn, Hans-Jürgen**
**Rehbocks Ecke 4**
**D-3550 Marburg/Lahn (DE)**
Erfinder : **Zimmermann, Josef**
**Auf der Krautweide 11**
**D-6231 Sulzbach (DE)**

Jouve, 18, rue St-Denis, 75001 Paris, France

EP 0 063 343 B1

## Beschreibung

Die Erfindung bezieht sich auf ein nadelloses Injektionsgerät mit einer Kolbenpumpe für das zu injizierende Medium, die mit einem Antriebsmotor verbunden ist, dessen Arbeitskolben verschiebbar in einer zylindrischen Bohrung des Motorgehäuses angeordnet ist, wobei der Antriebsmotor ein gegen Verdrehen gesichertes Federgehäuse aufweist, in dem eine Arbeitsfeder angeordnet ist, und das Federgehäuse mit einer Ausnehmung zur Aufnahme einer im Motorgehäuse angeordneten Klinke versehen ist.

Nach der US-Patentschrift 3 526 225 ist ein nadelloses Injektionsgerät bekannt, bei dem der Antriebsmotor aus einem Kolben mit kurzer Kolbenstange, einer Feder sowie einer verstellbaren Führung für die Feder besteht. Die Feder wird hydraulisch zusammengepreßt und durch die Hydraulikflüssigkeit in dieser Position gehalten. Die Führung für die Feder steht mit einer Spindel in Eingriff, über die der Abstand der freien Endfläche der Führung zur Stirnfläche der Kolbenstange und damit der Hub der Kolbenstange eingestellt werden kann. Nachteilig ist, daß die zusammengepreßte Feder durch die Hydraulikflüssigkeit in ihrer Lage gehalten werden muß und zum Entspannen der Abzugshebel krampfhaft festgehalten werden muß, damit die Hydraulikflüssigkeit ohne Bremswirkung für die Feder aus dem Hydraulikzylinder auslaufen kann.

Nach der US-Patentschrift 2 653 602 ist ein nadelloses Injektionsgerät gemäß Oberbegriff des Patentanspruchs 1 bekannt, das einen Antriebsmotor aufweist, der mit einer Kolbenpumpe fest verbunden ist. Die Impfstoffpumpe ist mit einem Federgehäuse verbunden. Das Federgehäuse ist verschiebbar in einer zylindrischen Bohrung im Motorgehäuse gelagert und weist eine Ausnehmung zur Aufnahme einer Klinke auf. Die Klinke wird durch einen als Klinke ausgebildeten Hebel unterstützt, der mittels eines schwenkbaren Stiftes gegen Verdrehen gesichert ist. Nachteilig ist das komplizierte Bedienen und Entsichern des Unterstützungshebels für das zwei Hände gleichzeitig erforderlich sind. Ferner ist nicht gewährleistet, daß das Auslösen der Kolbenpumpe nur bei Hautkontakt des Injektionsgerätes möglich ist.

Hier will die Erfindung Abhilfe schaffen. Die Erfindung, wie sie in den Ansprüchen gekennzeichnet ist, löst die Aufgabe dadurch, daß die Arbeitsfeder sich einerseits auf einer Stützfläche im Federgehäuse und andererseits auf einer Stirnfläche, die die Bohrung im Motorgehäuse begrenzt, abstützt und von einem als Kolbenstange ausgebildeten Teil des Arbeitskolbens geführt wird, das mit einer Kurbel und einem Gewinde versehen ist, das mit einem entsprechenden Gewinde im Federgehäuse im Eingriff steht, daß die Klinke durch einen im Motorgehäuse verschiebbar angeordneten Riegel unterstützt wird, der mit einem konischen Teil versehen ist, auf den sich die Klinke beim Einrasten in die Ausnehmung des Federgehäuses aufschiebt, und daß ein Ende des Riegels mit einem Bedienungselement für den Antriebsmotor starr verbunden ist.

Das andere im Motorgehäuse freibewegliche Ende des Riegels ist mit einem Zapfen versehen, der in einem im Motorgehäuse befestigten Lager geführt ist und eine Feder trägt, die sich auf dem Lager und dem freien Ende des Zapfens abstützt.

Die durch die Erfindung erreichten Vorteile sind im wesentlichen darin zu sehen, daß sichergestellt ist, daß der Arbeitskolben und damit die Kolbenpumpe erst dann in Bewegung gesetzt werden kann, d. h. der Schuß erst dann ausgelöst wird, wenn das Injektionsgerät z. B. auf die Haut, also auf die Stelle, an der die Injektion vorgenommen werden soll, aufgedrückt wird. Der Schuß läuft nach Auslösen automatisch ab und die Injektion erfolgt mit Hautkontakt. Bedienungsfehler sind somit ausgeschlossen.

Im folgenden wird die Erfindung an Hand von lediglich einen Ausführungsweg darstellenden Zeichnungen näher erläutert. Es zeigt :

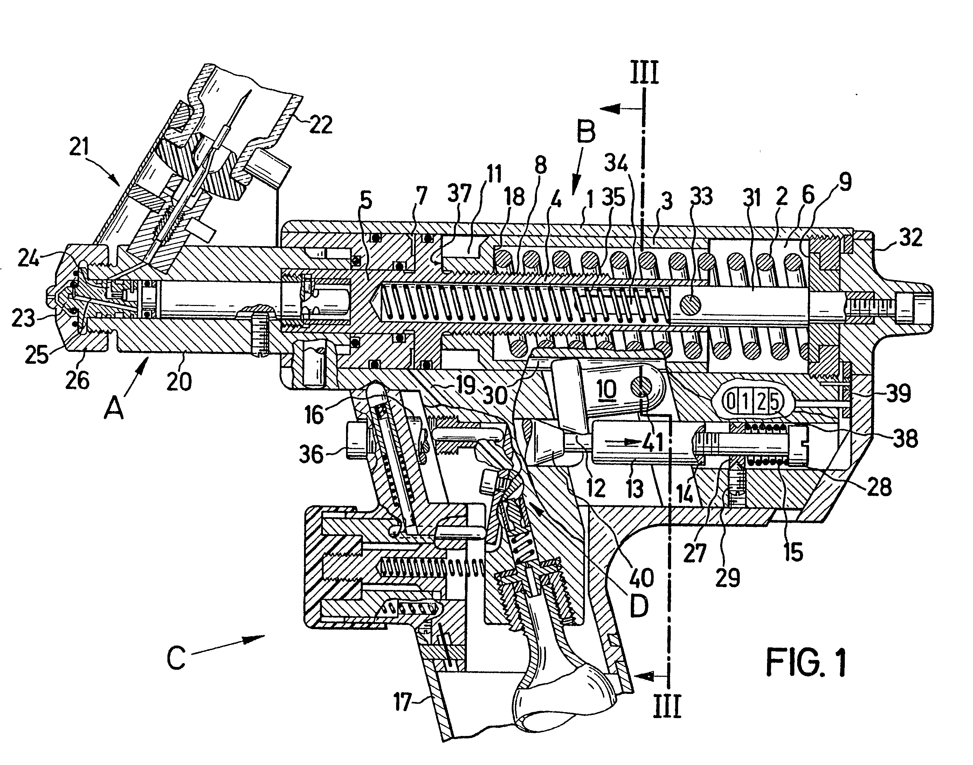

Figur 1 eine Seitenansicht des Injektionsgerätes teilweise und in verschiedenen Ebenen geschnitten in abgeschossenem Zustand,

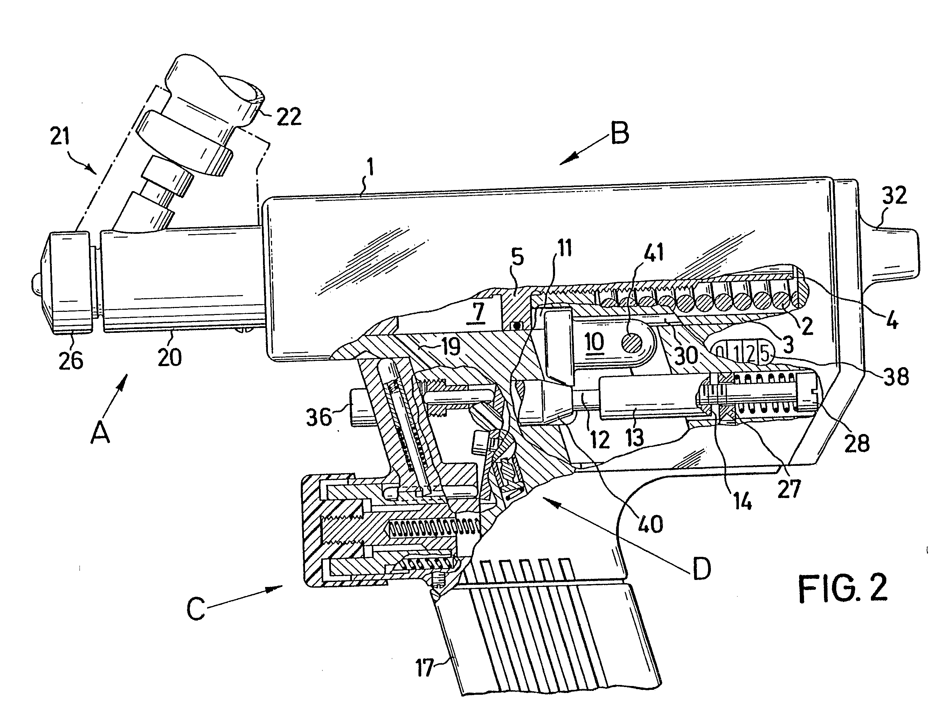

Figur 2 eine Seitenansicht des Injektionsgerätes teilweise und in verschiedenen Ebenen geschnitten in geladenem Zustand.

Figur 3 den Schnitt III-III der Figur 1.

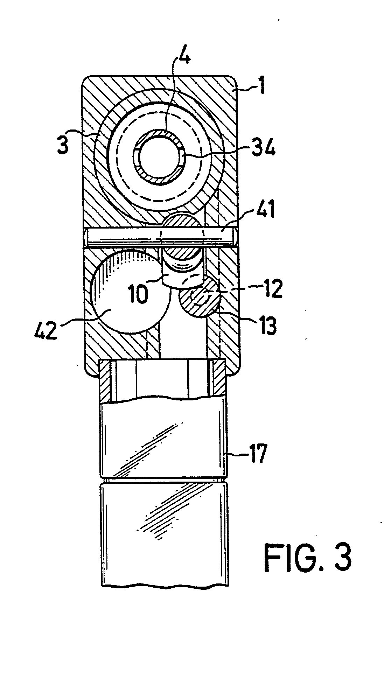

Das nadellose Injektionsgerät besteht im wesentlichen aus einer Kolbenpumpe (A) für das zu injizierende Medium, die mit einem Antriebsmotor (B) lösbar verbunden ist. Das Gehäuse (20) der Kolbenpumpe (A) trägt eine Einrichtung (21) zur Aufnahme eines Behälters (22) für das zu injizierende Medium sowie einen Ventilkörper (23) mit Ein- und Auslaßkanal (24) und (25) für das zu injizierende Medium und eine Düse (26). Der Arbeitskolben (5) des Antriebsmotors (B) ist in einer zylindrischen Bohrung (6) des Motorgehäuses (1) angeordnet. Der Antriebsmotor (B) weist ein durch eine Nut (30) gegen Verdrehen gesichertes Federgehäuse (3) auf, das mit einem Gewinde versehen ist. Dieses steht mit einem entsprechenden Gewinde (8) in Eingriff, das der als Kolbenstange (4) ausgebildete Teil des Arbeitskolbens (5) aufweist. Die Kolbenstange (4) ist mit einer Kurbel bestehend z. B. aus einem Mitnehmer (31) mit einem von außen zugänglichen Drehknopf (32) verbunden. Der Mitnehmer (31) weist einen Dorn (33) auf, der in einer Ausnehmung (34) in der Kolbenstange (4) geführt wird. Die Feder (35) dient der Montage des Mitnehmers (31). Durch Verdrehen des Drehknopfs (32) wird der Abstand zwischen dem Arbeitskolben (5) und der Schulter (37) des Federgehäuses (3) verstellt, wobei sich der Hubweg des Arbeitskolbens (5) und damit auch der der angekuppelten Kolbenpumpe (A) verändert. Dabei bleibt jedoch die maximale Pressung der Arbeitsfeder (2) unverändert, so daß bei unterschiedlicher Dosierung des Impfstoffs die Federkraft nicht geändert wird. Der Drehknopf (32) ist mit einem Zählwerk (38), gegebenenfalls über ein entsprechendes Getriebe (39) gekoppelt, welches die eingestellte Dosiermenge anzeigt. Das Zählwerk ist in einer Ausnehmung (42) (Figur 3) angeordnet.

Im Federgehäuse (3) ist eine Arbeitsfeder (2) angeordnet, die sich einerseits auf einer Stützfläche (18) im Federgehäuse (3) und andererseits auf einer Stirnfläche (9) abstützt, die die Bohrung (6) im Motorgehäuse (1) begrenzt. Das Federgehäuse (3) ist ferner mit einer Ausnehmung (11) zur Aufnahme einer im Motorgehäuse (1) um den Achsstift (41) drehbar angeordneten Klinke (10) versehen, die durch einen Riegel (13) unterstützt wird. Der Riegel (13) ist im Motorgehäuse (1) axial verschiebbar angeordnet und wird, sobald die Arbeitsfeder (2) gespannt ist, am freien Ende (14) von einer vorgespannten Feder (15) in Richtung des Pfeiles gezogen. Hierdurch wird die Klinke (10), die auf der Schulter (12) des Riegels (13) ruhte, durch den konischen Teil (40) des Riegels (13) angehoben und in die Ausnehmung (11) des Federgehäuses (3) geschoben. Die Feder (15) stützt sich auf einem Lagerring (27) für den Riegel (13) und dem freien Ende eines Zapfens (28), z. B. dem Knopf einer Schraube ab, den das freie Ende des Riegels (13) aufweist. Der Lagerring (27) ist im Motorgehäuse (1) mit Schraube (29) gehalten. Das Ende (16) des Riegels (13) ist mit Bedienungselement (C), z. B. einem Griff (17), z. B. mittels Schraube (36) fest verbunden, der sich somit ebenfalls gegenüber dem Motorgehäuse (1) verschieben läßt.

Zum Spannen der Arbeitsfeder (2) wird der Raum (7) mit einem Druckmittel z. B. mit Druckgas gefüllt, das über Kanal (19) zugeführt wird. Die Druckkraft des Arbeitskolbens (5) wirkt über das Federgehäuse (3) auf die Arbeitsfeder (2) und drückt diese zusammen, bis das Federgehäuse (3) an der Stirnfläche (9) anliegt. Die Klinke (10) kann nun in die Ausnehmung (11) des Federgehäuses (3) einrasten. Der mit Druckmittel gefüllte Raum (7) wird über einen nicht dargestellten Kanal entleert.

Zum Entspannen der Arbeitsfeder (2) wird der Griff (17) durch Druck der Düse (26) auf das zu injizierende Objekt gegen die Kraft der Feder (15) in Richtung Objekt geschoben. Dabei wird der Riegel (13) mitgenommen, wodurch die Klinke (10) ihre Stütze verliert ; die Arbeitsfeder (2) kann sich entspannen.

(D) deutet die Steuerung für das Druckmittel an.

## Patentansprüche

1. Nadelloses Injektionsgerät mit einer Kolbenpumpe (A) für das zu injizierende Medium, die mit einem Antriebsmotor (B) verbunden ist, dessen Arbeitskolben (5) verschiebbar in einer zylindrischen Bohrung (6) des Motorgehäuses (1) angeordnet ist, wobei der Antriebsmotor (B) ein gegen Verdrehen gesichertes Federgehäuse (3) aufweist, in dem eine Arbeitsfeder (2) angeordnet ist, und das Federgehäuse (3) mit einer Ausnehmung (11) zur Aufnahme einer im Motorgehäuse (1) angeordneten Klinke (10) versehen ist, dadurch gekennzeichnet, daß die Arbeitsfeder (2) sich einerseits auf einer Stützfläche (18) im Federgehäuse (3) und andererseits auf einer Stirnfläche (9), die die Bohrung (6) im Motorgehäuse (1) begrenzt, abstützt und von einem als Kolbenstange (4) ausgebildeten Teil des Arbeitskolbens (5) geführt wird, das mit einer Kurbel (31, 32, 33) und einem Gewinde (8) versehen ist, das mit einem entsprechenden Gewinde im Federgehäuse (3) im Eingriff steht, daß die Klinke (10) durch einen im Motorgehäuse (1) verschiebbar angeordneten Riegel (13) unterstützt wird, der mit einem konischen Teil (40) versehen ist, auf den sich die Klinke (10) beim Einrasten in die Ausnehmung (11) des Federgehäuses (3) aufschiebt, und daß ein Ende (16) des Riegels (13) mit einem Bedienungselement (C) für den Antriebsmotor (B) starr verbunden ist.

2. Injektionsgerät nach Anspruch 1, dadurch gekennzeichnet, daß das andere Ende (14) des Riegels (13) mit einem Zapfen (28) versehen ist, der in einem im Motorgehäuse (1) befestigten Lager (27) geführt ist und eine Feder (15) trägt, die sich auf dem Lager und dem freien Ende des Zapfens (28) abstützt.

3. Injektionsgerät nach Anspruch 1, dadurch gekennzeichnet, daß die Kurbel mit einem Zählwert (38) zur Anzeige des Kolbenpumpenhubs verbunden ist.

## Claims

1. A needle-less injection instrument with a piston pump (A) for the medium to be injected, which is connected to a drive motor (B), the working piston (5) of which is arranged displaceably in a cylindrical bore 6 of the motor housing (1) the drive motor (B) having spring housing (3) which is secured against rotation and in which is located a working spring (2) and the spring housing (3) is provided with a recess (11) for receiving a pawl (10), wherein the working spring (2) is supported at one end on a supporting face (18) in the spring housing (3) and at the other end on an end face (9) limiting the bore (6) in the motor housing (1) and is guided by a part of the working piston (5) which is designed as a piston rod (4) which is provided with a crank (31, 32, 33) and a thread (8) engaging with a corresponding thread in the spring housing (3), the pawl (10) is supported by a bolt (13) arranged dispaceably in the motor housing (1), and provided with a conical part (40) onto which the pawl (10) is pushed when it engages into the recess (11) of the spring housing (3), and an end (16) of the bolt (13) is connected to an operating element (C) for the drive motor (B).

2. Injection instrument as claimed in claim 1, wherein the other end (14) of the bolt (13) is provided with a stud (28) which is guided in a bearing (27) fastened in the motor housing (1) and which carries a spring (15) supported on the bearing and on the free end of the stud (28).

3. Injection instrument as claimed in claim 1, wherein the crank is connected to a counter (38) for indicating the stoke of the piston pump.

## Revendications

1. Injecteur sans aiguille comprenant une pompe à piston (A) pour la substance à injecter, qui est accouplée à un moteur d'entraînement (B) dont le piston de travail (5) coulisse dans un alésage cylindrique (6) du corps de moteur (1), le moteur (B) possédant un barillet (3) empêché de tourner et contenant un ressort de travail (2), de même qu'une encoche (11), où vient s'encliqueter un cliquet (10) monté dans le corps de moteur (1), caractérisé en ce que le ressort de travail (2) s'appuie d'un côté sur une surface d'appui (18) dans le barillet (3) et de l'autre sur une face extrême (9) de l'alésage (6) du corps de moteur (1) et est guidé par une partie en forme de tige de piston (4) du piston de travail (5), laquelle est pourvue d'un dispositif de réglage (31, 32, 33) et d'un filetage (8), en prise avec un filetage correspondant dans le barillet (3), que le cliquet (10) est appuyé par un pêne (13) monté coulissant dans le corps de moteur (1) et doté d'une partie conique (40), sur laquelle monte, en glissant, le cliquet (10) lors de son encliquetage dans l'encoche (11) du barillet (3), et qu'une extrémité (16) du pêne est reliée rigidement à un élément de commande (C) du moteur (B).

2. Injecteur selon la revendication 1, caractérisé en ce que l'autre extrémité (14) du pêne (13) est dotée d'un tenon (28) qui est guidé dans un palier (27) fixé dans le corps de moteur (1) et qui porte un ressort (15) s'appuyant sur le palier et sur l'extrémité libre du tenon (28).

3. Injecteur selon la revendication 1, caractérisé en ce que le dispositif de réglage est couplé à un dispositif compteur (38) pour indiquer la course de la pompe à piston.

FIG. 1

0 063 343

FIG. 2

0 063 343

FIG. 3